(19) Europäisches Patentamt
European Patent Office
Office européen des brevets

(11) Publication number: **0 419 103 B1**

# EUROPEAN PATENT SPECIFICATION

(45) Date of publication of patent specification: **12.04.95**

(51) Int. Cl.⁶: **A61B 5/024**

(21) Application number: **90309870.5**

(22) Date of filing: **10.09.90**

(54) **Pulsimeter.**

(30) Priority: **13.09.89 JP 238084/89**

(43) Date of publication of application:
**27.03.91 Bulletin 91/13**

(45) Publication of the grant of the patent:
**12.04.95 Bulletin 95/15**

(84) Designated Contracting States:
**CH DE FR GB LI**

(56) References cited:
**EP-A- 0 005 949**
**EP-A- 0 117 330**
**US-A- 4 807 639**

(73) Proprietor: **SEIKO INSTRUMENTS INC.**
**31-1, Kameido 6-chome**
**Koto-ku**
**Tokyo 136 (JP)**

(72) Inventor: **Masaki, Hiroyuki**
**c/o Seiko Instruments Inc.,**
**31-1 Kameido 6-chome**
**Koto-ku,**
**Tokyo (JP)**

(74) Representative: **Sturt, Clifford Mark et al**
**J. MILLER & CO.**
**34 Bedford Row**
**Holborn**
**London WC1R 4JH (GB)**

**Description**

The present invention relates to a pulsimeter, which is designed to detect a heart beat and display a heart rate per unit time.

One type of conventional pulsimeter is designed to display only a heart rate per unit time, while another type of conventional pulsimeter is designed to display a heart heat index which shows an extent to which a subject's heart rate has increased in a range from 0 to a present target value for the heart rate.

Since the prior art is designed to display a heart rate per unit time or a level to which the currently measured heart rate has increased in a range from 0 to a target value, it is insufficient accurately to display in the form of a numerical value a heart beat function adapted with respect to each individual subject, who has his/her own range for heart rate.

It is an object of the present invention to provide a pulsimeter, which overcomes this disadvantage.

US-A-4,807,639 discloses a pulsimeter which detects a heart beat per unit time and comprises means for setting an upper limit and a normal value for the heart rate. It also comprises means for computing recovery rate - which it displays.

It is another object of the present invention, in its preferred form, to provide a pulsimeter which is able to detect a heart beat and to display a heart rate per unit time and an exercise intensity.

According to the present invention, there is provided a pulsimeter comprising: heart beat detecting means for detecting a heart beat and for generating a detected heart beat signal, heart rate computing means for computing a heart rate per unit time on the basis of the detected heart beat signal, setting means for setting an upper limit value for the heart rate and a normal value for the heart rate, and display means for displaying an output, characterised in that exercise intensity computing means are provided for computing exercise intensity on the basis of output data from said heart rate computing means and said setting means; said exercise intensity computing means being arranged to compute the exercise intensity on the basis of the following equation:

$$\text{Exercise intensity} = \frac{(\text{computed heart rate - normal value})}{(\text{upper limit value - normal value})} \times 100(\%)$$

$$\ldots\ldots (1)$$

and said display means serves to display said exercise intensity.

By virtue of the above described arrangement, it is possible to display an exercise intensity that shows a condition level for a subject's present heart beat function within a particular heart rate range, which varies from person to person.

Preferably, the display means is arranged to display the heart rate as well as the exercise intensity.

The invention is described further, by way of example, with reference to the accompanying drawings, in which:-

Figure 1 is a block diagram of a pulsimeter according to the present invention;

Figure 2 is a flow chart representing a process performed by exercise intensity computing means of the pulsimeter for computing an exercise intensity;

Figure 3 schematically shows a hardware arrangement for one embodiment of the pulsimeter according to the present invention; and

Figure 4 shows one example of a display of the pulsimeter of Figure 3.

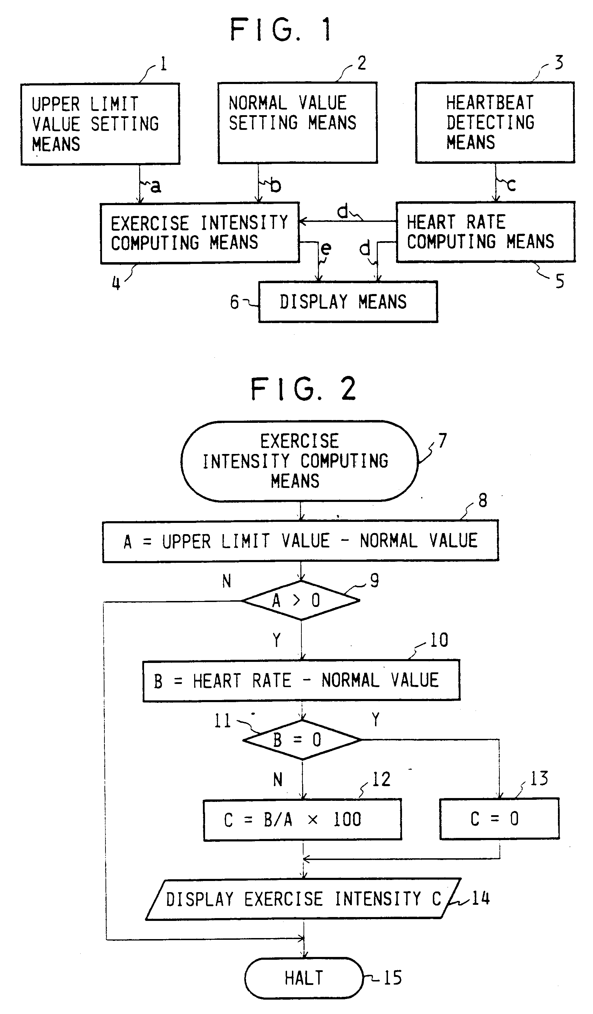

Figure 1 is a functional block diagram of a pulsimeter according to the present invention comprising external input means (not shown), for example a KEY input unit, upper limit value setting means 1, which stores a numerical value input as an upper limit value for heart rate, and normal value setting means 2, which similarly stores a numerical value input as a normal value. Heart beat detecting means 3 either electrically detects a muscular potential of the heart or photo-electrically detects a blood stream flowing through a blood vessel to output a detected heart beat signal $\underline{c}$ to heart rate computing means 5, which computes a heart rate per unit time (one minute in this embodiment) and outputs heart rate data $\underline{d}$. Exercise intensity computing means 4 is supplied with the upper limit value data $\underline{a}$ and normal value data $\underline{b}$, which are output respectively from the upper limit value setting means 1 and normal value setting means 2, together with the heart rate data $\underline{d}$, to compute an exercise intensity and to output exercise intensity data $\underline{e}$.

Display means 6 displays the heart rate data $d$, exercise intensity data $e$ and other information, for example time.

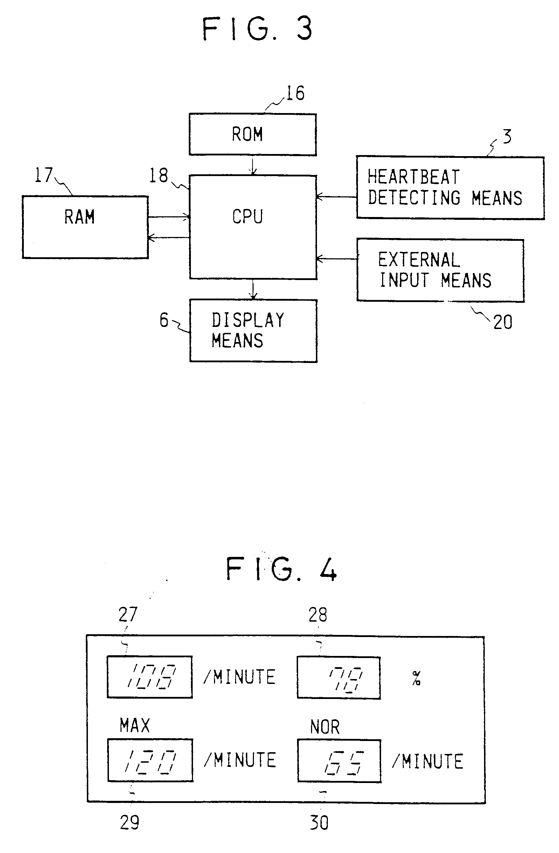

Figure 3 is a block diagram showing the hardware arrangement of this embodiment when realised by a program (software) using, for example, a one-chip micro-computer. The arrangement comprises an external input member 20, e.g. a KEY, the heart beat detecting means 3, a CPU (logical processing circuit) 18 which executes processing operations, for example, computation of a heart rate per unit time and an exercise intensity from the detected heart beat signal $c$, a RAM (random access memory) 17 which stores various data, a ROM (read only memory) 16 in which is stored each operation process in the form of a program, and the display means 6. Figure 4 shows an example of a display of the pulsimeter according to this embodiment, in which are displayed a heart rate per minute 27, a set upper limit value 29 and a set normal value 30 for heart rate, and an exercise intensity 28.

A specific operation will now be explained. A person who is a subject for measurement sets an upper limit value and a normal value for heart rate through the external input member 20 in advance of the measurement of his or her heart beat. The upper limit value is either a value which is used as a level for judging it dangerous for the subject's heart rate to exceed this value, or a target value for heart rate during exercise, and is set personally by the subject. The normal value is a value for heart rate at the time when the subject is not taking exercise, i.e. when he or she is at rest. In this embodiment, the upper limit value and normal value are exemplarily set at 120/minute and 65/minute, respectively. These input numerical values are stored at pre-determined addresses in the RAM 17. When the subject starts a heart beat measurement by actuating the external input means 20 after inputting the upper limit value and the normal value, the heart beat detecting means 3 detects, for example, blood flow by the use of an optical sensor, and converts it into an electrical signal and then outputs to the CPU 18 the detected heart beat signal $c$, which is synchronised with the heart beat. On receiving the detected heart beat signal $c$, the CPU 18 calculates a time elapsed after the previous heart beat detecting signal and computes a heart rate per minute according to a heart beat rate computing program stored in the ROM 16 and then outputs the obtained heart rate to the RAM 17 and the display means 6. In addition, the CPU 18 computes an exercise intensity, which is given by the equation (1), according to an exercise intensity computing program similarly stored in the ROM 16.

Figure 2 is a flow chart representing the process for computing an exercise intensity. When an exercise intensity computing operation is started (step 7), the upper limit value and the normal value, stored in the RAM 17, are read out to make a calculation (step 8), i.e. the upper limit value minus the normal value. Assuming that "A = upper limit value - normal value", if $A \leq 0$, neither calculation nor display of an exercise intensity is executed, and the process returns to HALT (step 15). If $A > 0$, then the normal value is subtracted from a heart rate that is computed according to the heart beat computing program (step 10). Assuming that "B = heart rate - normal value", if B = 0, the exercise intensity C is 0 and hence C is determined to be 0 (step 13). Then, the exercise intensity C is displayed (step 14) and the process returns to HALT (step 15). If $B \neq 0$, C is obtained from A and B (step 12), which have already been computed, and the exercise intensity C is displayed (step 14). Then, the process returns to HALT (step 15). More specifically, if the heart rate is, for example, 108/minute, and the upper limit value and the normal value have previously been set at 120/minute and 65/minute, as described above, the exercise intensity C is determined to be 78 (%) from the equation (1) and displayed, as exemplarily shown in Figure 4.

These calculations are executed to obtain an exercise intensity every time the detected heart beat signal $c$ is input, and the heart rate data is calculated and displayed. Since the computing time is several tens of msec, the heart rate and the exercise intensity are seen to change simultaneously on the display of the pulsimeter, thus, the heart beat condition can be grasped in real time.

As has been described above, it is possible to grasp a heart beat condition in real time and with a level which is suitable for each individual subject's body, by providing upper limit value setting means and normal value setting means and by computing an exercise intensity from a measured heart rate with exercise intensity computing means and further displaying the exercise intensity.

For example, when the exercise intensity is minus, it shows that the heart rate is lower than normal; when the exercise intensity is 0, it shows that the heart rate is normal. When the heart rate rises during exercise, the extent to which the heart rate rises in the range from the normal value to the upper limit value is expressed directly as a numerical value. Accordingly, the exercise intensity can be employed as a standard for the quantity of exercise.

When the heart beat is measured after the subject has done a pre-determined quantity of exercise, an exercise intensity is simultaneously displayed. It is, therefore, possible to check instantly the heart beat function and to obtain the required data, and the exercise intensity can also be utilised as a barometer indicative of the condition of one's health.

EP 0 419 103 B1

## Claims

1. A pulsimeter comprising: heart beat detecting means (3) for detecting a heart beat and for generating a detected heart beat signal, heart rate computing means (5) for computing a heart rate per unit time on the basis of the detected heart beat signal, and setting means (1,2) for setting an upper limit value for the heart rate and a normal value for the heart rate, and display means (6) for displaying an output; characterised in that exercise intensity computing means (4) are provided for computing exercise intensity on the basis of output data from said heart rate computing means and said setting means; said exercise intensity computing means being arranged to compute the exercise intensity on the basis of the following equation:

$$\text{Exercise intensity} = \frac{\text{(computed heart rate - normal value)}}{\text{(upper limit value - normal value)}} \times 100\ (\%)$$

and said display means serves to display said exercise intensity.

2. A pulsimeter according to claim 1, characterised in that said exercise intensity computing means is arranged to compute said exercise intensity every time said detected heart beat signal is supplied from said heart beat detecting means to said heart rate computing means.

3. A pulsimeter according to claim 1 or 2, characterised in that said setting means comprises first setting means (1) for setting said upper limit value and second setting means (2) for setting said normal value.

4. A pulsimeter according to any of claims 1 to 3 characterised by an external input member for inputting said upper limit value and said normal value.

5. A pulsimeter according to any of claims 1 to 4 characterised in that said display means is arranged to display also said computed heart rate.

## Patentansprüche

1. Pulsmesser umfassend: ein Herzschlagerfassungsmittel (3) zum Erfassen eines Herzschlags und zum Erzeugen eines dem erfaßten Herzschlag entsprechenden Signals, ein Herzfrequenzberechnungsmittel (5) zum Berechnen einer Herzfrequenz pro Zeiteinheit auf der Basis des dem erfaßten Herzschlag entsprechenden Signals und ein Setzmittel (1,2) zum Setzen eines oberen Grenzwerts für die Herzfrequenz und eines Normalwerts für die Herzfrequenz und ein Anzeigemittel (6) zum Anzeigen einer Ausgabe; dadurch gekennzeichnet, daß ein Übungsintensitätsberechnungsmittel (4) zum Berechnen einer Übungsintensität auf der Basis von Ausgabedaten von dem Herzfrequenzberechnungsmittel und dem Setzmittel bereitgestellt ist; wobei das Übungsintensitätsberechnungsmittel dazu ausgebildet ist, die Übungsintensität auf der Basis folgender Gleichung:

$$\text{Übungsintensität} = \frac{\text{(berechnete Herzfrequenz - Normalwert)}}{\text{(oberer Grenzwert - Normalwert)}} \times 100\ (\%)$$

zu berechnen, und das Anzeigemittel dazu dient, die Übungsintensität anzuzeigen.

2. Pulsmesser nach Anspruch 1, dadurch gekennzeichnet, daß das Übungsintensitätsberechnungsmittel dazu ausgebildet ist, die Übungsintensität jedesmal zu berechnen, wenn das Herzschlagerfassungsmittel das dem erfaßten Herzschlag entsprechende Signal an das Herzfrequenzberechnungsmittel gibt.

3. Pulsmesser nach Anspruch 1 oder 2, dadurch gekennzeichnet, daß das Setzmittel ein erstes Setzmittel (1) zum Setzen des oberen Grenzwerts und ein zweites Setzmittel (2) zum Setzen des Normalwerts umfaßt.

4

**4.** Pulsmesser nach einem der Ansprüche 1 bis 3, gekennzeichnet durch ein Element für externe Eingabe zum Eingeben des oberen Grenzwerts und des Normalwerts.

**5.** Pulsmesser nach einem der Ansprüche 1 bis 4, dadurch gekennzeichnet, daß das Anzeigemittel dazu ausgebildet ist, auch die berechnete Herzfrequenz anzuzeigen.

**Revendications**

**1.** Pulsomètre comprenant : des moyens (3) de détection de battements de coeur destinés à détecter un battement de coeur et à générer un signal de battements de coeur détectés, des moyens (5) de calcul de la fréquence cardiaque destinés à calculer une fréquence cardiaque par unité de temps sur la base du signal de battements de coeur détectés, des moyens (1, 2) de fixation destinés à fixer une valeur limite supérieure de la fréquence cardiaque et une valeur normale de la fréquence cardiaque, et des moyens (6) d'affichage destinés à affiche, une grandeur de sortie ; caractérisé en ce que des moyens (4) de calcul de l'intensité de l'effort sont prévus pour calculer l'intensité de l'effort sur la base de données de sortie provenant desdits moyens de calcul de la fréquence cardiaque et desdits moyens de fixation; ces moyens de calcul de l'intensité de l'effort étant prévus pour calculer l'intensité de l'effort par l'équation suivante :

$$\text{Intensité de l'effort} = \frac{(\text{fréquence cardiaque calculée - valeur normale})}{(\text{valeur limite supérieure - valeur normale})} \times 100\ (\%)$$

et les moyens d'affichage servent à afficher l'intensité de l'effort.

**2.** Pulsomètre suivant la revendication 1, caractérisé en ce que les moyens de calcul de l'intensité de l'effort sont prévus pour calculer l'intensité de l'effort chaque fois que le signal de battements de coeur détectés est fourni par les moyens de détection de battements de coeur aux moyens de calcul de la fréquence cardiaque.

**3.** Pulsomètre suivant la revendication 1 ou 2, caractérisé en ce que les moyens de fixation comprennent des premiers moyens (1) de fixation destinés à fixer la valeur limite supérieure et des seconds moyens (2) de fixation destinés à fixer la valeur normale.

**4.** Pulsomètre suivant l'une quelconque des revendications 1 à 3, caractérisé par un élément extérieur d'introduction destiné à introduire la valeur limite supérieure et la valeur normale.

**5.** Pulsomètre suivant l'une quelconque des revendications 1 à 4, caractérisé en ce que lesdits moyens d'affichage sont prévus pour afficher aussi la fréquence cardiaque calculée.

# FIG. 1

| UPPER LIMIT VALUE SETTING MEANS | 1 | NORMAL VALUE SETTING MEANS | 2 | HEARTBEAT DETECTING MEANS | 3 |

```
UPPER LIMIT         NORMAL VALUE        HEARTBEAT
VALUE SETTING       SETTING MEANS       DETECTING
MEANS                                   MEANS
   1                    2                   3

        a               b                       c

 EXERCISE INTENSITY   ←───d───   HEART RATE
 COMPUTING MEANS                 COMPUTING MEANS

   4         e       d                        5

         6   DISPLAY MEANS
```

# FIG. 2

```
        ┌──────────────────────┐
        │      EXERCISE        │ 7
        │ INTENSITY COMPUTING  │
        │      MEANS           │
        └──────────────────────┘

 ┌──────────────────────────────────────┐ 8
 │ A = UPPER LIMIT VALUE − NORMAL VALUE  │
 └──────────────────────────────────────┘

   N          ◇ A > 0 ◇  9

              Y

 ┌──────────────────────────────────────┐ 10
 │   B = HEART RATE − NORMAL VALUE       │
 └──────────────────────────────────────┘

 11    ◇ B = 0 ◇     Y

   N              12                13
 ┌──────────────┐        ┌──────────┐
 │ C = B/A × 100│        │  C = 0   │
 └──────────────┘        └──────────┘

 ┌────────────────────────────────────┐ 14
 │ DISPLAY EXERCISE INTENSITY C        │
 └────────────────────────────────────┘

          ( HALT )  15
```

$A = $ UPPER LIMIT VALUE $-$ NORMAL VALUE

$A > 0$

$B = $ HEART RATE $-$ NORMAL VALUE

$B = 0$

$C = B/A \times 100$

$C = 0$

DISPLAY EXERCISE INTENSITY C

HALT

# FIG. 3

```
                          16
                  ┌───────────┐
                  │    ROM    │
                  └───────────┘                              3
                        │
 17          18         ▼                          ┌──────────────────┐
┌──────┐        ┌───────────┐                      │ HEARTBEAT        │
│ RAM  │───────▶│           │◀─────────────────────│ DETECTING MEANS  │
│      │◀───────│    CPU    │                      └──────────────────┘
└──────┘        │           │
                │           │◀─────────────────────┌──────────────────┐
                └───────────┘                      │ EXTERNAL         │
                      │                             │ INPUT MEANS      │
                      ▼                             └──────────────────┘
              ┌───────────┐                                      20
         6    │ DISPLAY   │
              │ MEANS     │
              └───────────┘
```

# FIG. 4

```
   27                    28
┌─────────────────────────────────────────────────────────────┐
│  ┌──────┐                  ┌──────┐                           │
│  │ 109  │ /MINUTE          │  78  │   %                       │
│  └──────┘                  └──────┘                           │
│                                                               │
│    MAX                       NOR                              │
│  ┌──────┐                  ┌──────┐                           │
│  │ 120  │ /MINUTE          │  55  │ /MINUTE                   │
│  └──────┘                  └──────┘                           │
└─────────────────────────────────────────────────────────────┘
   29                    30
```

7